# EUROPÄISCHE PATENTSCHRIFT

(11) **EP 1 040 788 B1**
(45) Veröffentlichungstag und Bekanntmachung des Hinweises auf die Patenterteilung: **19.12.2001**
(21) Anmeldenummer: 99890109.4
(22) Anmeldetag: 29.03.1999
(51) Int. Cl.: A61B 5/00, G01N 21/64

(54) **Vorrichtung zur quantitativen Bestimmung der örtlichen Verteilung einer Messgrösse**
Apparatus for quantitative determination of the local distribution of a measurement value
Dispositif pour la détermination quantative de la distribution locale d'une grandeur a mesurer

(43) Veröffentlichungstag der Anmeldung: 04.10.2000
(73) Patentinhaber: F.HOFFMANN-LA ROCHE AG, 4070 Basel (CH)
(72) Erfinder: Hartmann, Paul, 8160 Weiz (AT); Ziegler, Werner E., 8043 Graz (AT)
(74) Vertreter: Babeluk, Michael, Dipl.-Ing. Mag.

(56) Entgegenhaltungen:
- EP-A- 0 516 610
- DE-A- 3 900 191
- US-A- 4 005 700
- US-A- 4 041 932
- US-A- 4 259 963
- US-A- 5 593 899
- US-A- 5 643 681

## Beschreibung

Die Erfindung betrifft eine Vorrichtung zur quantitativen Bestimmung der örtlichen Verteilung einer Meßgröße, mit einem auf eine Meßoberfläche, beispielsweise eine Organoberfläche, einen Hautbereich oder eine Zellkultur, aufbringbaren flächigen Sensorfilm mit bekannten Diffusionseigenschaften für einen zu bestimmenden Parameter, wobei der Sensorfilm einen auf den zu bestimmenden Parameter mit einer Änderung zumindest einer optischen Eigenschaft reagierenden Lumineszenzindikator aufweist, mit einer Anregungs- und Detektionseinheit, welche eine Einrichtung zur Bereitstellung einer Anregungsstrahlung zumindest einer Wellenlänge und eine bildgebende Detektionseinrichtung, vorzugsweise eine CCD-Kamera aufweist, sowie mit einer Auswerteeinrichtung zur Aufbereitung der durch die Anregungs- und Detektionseinrichtung gewonnenen Bildinformation.

Für die medizinische Diagnostik sowie für die Überprüfung und Steuerung von medizinischen Therapieverfahren ist es oft von großer Bedeutung, die örtliche Verteilung der Konzentration einer Meßgröße an einer Organoberfläche, beispielsweise der Haut, oder auch die Verteilung der Durchflußrate einer bestimmten Substanz durch eine Grenzfläche, beispielsweise einer Organoberfläche, eines Hautbereiches oder auch einer Zellkultur in einer Laboranordnung zu bestimmen.

In diesem Zusammenhang ist aus der EP 0 516 610 B1 ein schematischer Aufbau bekannt geworden, mit welchem der Wert des Materieflusses, beispielsweise des Sauerstoffflusses, durch eine Grenzfläche, beispielsweise einem Hautbereich, gemessen werden kann. Die der Grenz- oder Meßfläche zuordenbare Sensorschicht der Meßvorrichtung setzt dem zu messenden Materiefluß einem bekannten oder vorbestimmbaren, endlichen Widerstand entgegen, wobei in der Sensorschicht zumindest ein optischer Indikator zur Bestimmung der Materiekonzentration auf einer Seite der Sensorschicht vorgesehen ist. Aus dem gemessenen bzw. bekannten Konzentrationswert auf der zweiten Seite der Sensorschicht bzw. der Differenz zum ersten Konzentrationswert wird der Materiefluß durch die Grenzfläche bestimmt. Die Sensorschicht kann gemäß einer Ausführungsvariante entweder durch eine Vielzahl von Detektoren oder auch durch ein bildgebendes System (z. B. CCD) flächenförmig abgetastet werden.

Aus der US 5 593 899 A ist eine Vorrichtung und ein Verfahren zur Messung der Sauerstoffversorgung der Haut bekannt geworden, wobei die von der Sauerstoffkonzentration abhängige Löschung eines Fluoreszenzindikators genützt wird. Zur Messung der Sauerstoffversorgung wird auf den entsprechenden Hautbereich eine lumineszierende Schicht als Bestandteil einer Hautcreme aufgetragen, und durch einen sauerstoffimpermeablen Film abgedeckt. Die in einem Kunststoffgehäuse angeordnete optische Einrichtung weist eine Plastik- oder Glasabdeckung auf, welche direkt auf den O₂-impermeablen Film aufgesetzt wird. Daran anschließend ist ein Interferenzfilter sowie eine Fotodiode angeordnet. Die lumineszierende Schicht wird über Lichtleiter von einer modulierten Strahlungsquelle mit Anregungsstrahlung beaufschlagt. Weiters sind im Kunststoffgehäuse Heizelemente angeordnet, welche mit einem Temperaturregelkreis in Verbindung stehen und den Meßbereich auf eine Temperatur zwischen 39° und 42°C erwärmen. Die beschriebene Anordnung eignet sich allerdings nur für eine integrale Messung über den gesamten von der optischen Einrichtung erfaßten Meßbereich. Genaue Aussagen, beispielsweise über Grenzbereiche zwischen ausreichend mit Sauerstoff versorgten bzw. unterversorgten Gebieten sind mit dieser Einrichtung allerdings nicht möglich.

Theoretische Überlegungen über die örtliche Verteilung des Sauerstofflusses bzw. der subcutanen Sauerstoffkonzentration und der Vorschlag für ein bildgebendes Meßverfahren sind weiters in dem Artikel "Fluorescence Lifetime Imaging of the Skin PO₂: Instrumentation and Results" aus "Advances in Experimental Medicine and Biologig, Vol. 428,P.605-611 (1997), Verlag Plenum Press N.Y. geoffenbart. In diesem Artikel wird eine Sensorfolie zur Messung der transcutanen Sauerstoffkonzentration beschrieben, welche eine der Hautoberfläche zugewandte optische Isolationsschicht, eine Sensorschicht mit einem Lumineszenzindikator mit O₂-sensitiver Abklingzeit und eine für Sauerstoff undurchlässige Trägerschicht aufweist. Eine ebenfalls beschriebene Membran zur Bestimmung des Sauerstoffflusses unterscheidet sich von der erstgenannten durch eine Diffusionsbarriere mit bekannter Sauerstoffdurchlässigkeit, welche anstelle der O₂-impermeablen Schicht angeordnet ist. Zur Messung wird die Sensorfolie auf die Meßoberfläche, beispielsweise einen Hautbereich, aufgebracht. Beim Meßverfahren bedient man sich einer Modulationstechnik, wobei die die Anregungsstrahlung in Richtung der Sensormenbran emittierenden LEDs durch einen Square-Wave-Generator angetrieben werden und eine rechteckförmig modulierte Anregungsstrahlung emittieren. Die von der Sensorfolie ausgehende, phasenverschobene Meßstrahlung wird von einer CCD-Kamera mit modulierter Verstärkung erfaßt und pixelweise an eine Rechnereinheit zur Bildverarbeitung weitergeleitet. Dokumentiert wird die in einer Polymerschicht gemessene Sauerstoffverteilung sowie die unterschiedliche Sauerstoffverteilung eines Hautbereichs.

Weitere bildgebende Meßverfahren unter Verwendung der Phasenfluorometrie sind aus der US 5 485 530 A bekannt geworden.

Schließlich ist aus Sensors and Actuators B 51(1998) 163-170 im Artikel "A modular luminescence lifetime imaging system for mapping oxygen distribution in biological samples" ein bildgebendes Verfahren zur Messung der zweidimensionalen Sauerstoffverteilung in planaren Optoden beschrieben, bei welchem in einer Versuchsanordnung in einer Testkammer mehrere Perfusionskanäle angeordnet sind, welche mit Wasser unterschiedlicher Sauerstoffkonzentration durchflossen werden. In Richtung des optischen Systems sind die Kanäle mit einer planaren Optode verschlossen, welche dicht an der Testkammer anliegt. Zur Anregung der planaren Optode dient eine faseroptische Ringlichtquelle, zur Erfassung der Meßstrahlung eine CCD-Kamera.

Aufgabe der vorliegenden Erfindung ist es, die eingangs erwähnte Vorrichtung derart weiterzubilden, daß damit auf einfache Weise reproduzierbare Messungen der örtlichen Verteilung der Konzentration einer Meßgröße auf einer ggf. gekrümmten Meßoberfläche bzw. die örtliche Verteilung des Materieflusses durch eine ggf. gekrümmte Meßoberfläche gemessen und bildlich dargestellt werden kann.

Diese Aufgabe wird erfindungsgemäß dadurch gelöst, daß die Vorrichtung einen auf die Meßoberfläche aufsetzbaren Applikatortubus aufweist, dessen der Meßoberfläche zugewandte Rand am Sensorfilm elastisch anliegt und für die Detektionseinrichtung störende Strahlung undurchlässig ist.

Ohne sich darauf einzuschränken wird die vorliegende Erfindung im folgenden anhand der Messung der örtlichen Verteilung der transcutanen Sauerstoffkonzentration sowie der örtlichen Verteilung des Sauerstoffflusses durch die Haut näher beschrieben, wobei die dargelegten Vorteile uneingeschränkt auf andere medizinische bzw. biologische Größen und Parameter anwendbar sind. Beispielsweise kann bei einer geeigneten Wahl des Lumineszenzindikators die subcutane CO₂-Konzentration bzw. die Verteilung des CO₂-Flusses durch die Haut gemessen werden. Mit der erfindungsgemäßen Vorrichtung kann auch die örtliche Verteilung der Ionenkonzentration bzw. des Ionenflusses gemessen werden, wenn der an der Meßoberfläche anbringbare Sensorfilm einen Lumineszenzindikator aufweist, der auf das entsprechende Ion reagiert.

Durch den auf die Meßoberfläche aufsetzbaren Indikatortubus wird insbesondere bei der medizinischen Anwendung eine eindeutig definierte optische Anordnung realisiert, welche reproduzierbare Meßergebnisse liefert. Neben einem, durch die vorgegebene Tubuslänge konstanten Abstand zur Meßoberfläche dient der Tubus mit seinen elastisch am Sensorfilm anliegenden Rand dazu, Fehllicht bzw. Störstrahlung von der empfindlichen Detektionseinheit fernzuhalten. Dies gilt auch für unebene Meßoberflächen, an welche sich der elastische Rand des Applikatortubus optimal anschmiegt.

Die Vorrichtung eignet sich besonders zur Anwendung an gekrümmten Meßoberflächen, beispielsweise bei der Messung der subcutanen Sauerstoffkonzentration im Bereich der Extremitäten einer Person, wobei die Detektionseinrichtung vorzugsweise eine Einheit zur Bestimmung der örtlichen Fluoreszenzabklingzeit oder einer davon abgeleiteten Größe aufweist.

Um bei einem bildgebenden Meßverfahren unabhängig von Inhomogenitäten der Belegungsdichte des Lumineszenzindikators im Sensorfilm und unabhängig vom inhomogenen optischen Verhältnissen zu werden, bedient sich die Erfindung bevorzugt der Abklingzeitmessung. Eine spezielle CCD-Kamera wird dabei so betrieben, daß die Information in jedem Pixel mit der Abklingzeit am entsprechenden, dem Pixel zugeordneten Meßort, in Beziehung gebracht werden kann. Durch die Messung eines nur von der Abklingzeit des Indikators bestimmten Parameters ergibt sich insbesondere der Vorteil, daß die Messung auch unabhängig von der lokalen Beleuchtungsstärke der Sensorfolie wird. Damit eröffnet sich die Möglichkeit, auch gekrümmte Oberflächen mit einer einfachen Anordnung der Anregungslichtquelle auszuleuchten, ohne für eine homogene Ausleuchtung sorgen zu müssen, solange an jedem Meßpunkt für die Abklingzeitmessung ausreichende Lumineszenzstrahlung erzeugt wird. Da die Abklingzeitmessung im Gegensatz zu einer Intensitätsmessung keiner Abhängigkeit vom Quadrat des Meßobjektabstandes unterliegt und unterschiedliche Laufzeiten des Lichtes verursacht durch unterschiedliche Distanzen des Meßobjektes bei den verwendeten Modulationsfrequenzen im Rahmen der Meßgenauigkeit vernachlässigbar sind, eignet sich die Abklingzeitmessung hervorragend für die Messung an Armen und Beinen oder anderen gekrümmten Hautflächen von Patienten.

Eine vorteilhafte Ausführungsvariante sieht vor, daß der Applikatortubus an dem der Meßoberfläche abgewandten, verschließbaren Ende eine Anschlußvorrichtung für die Anregungs- und Detektionseinheit aufweist.

Erfindungsgemäß kann der Applikatortubus am von der Meßoberfläche abgewandten Ende mit einer für die Anregungs- und Meßstrahlung durchlässigen Abdeckung, vorzugsweise einer Glasplatte abgeschlossen, oder mit einem abnehmbaren Deckel verschließbar sein. Dies ist insbesondere dann von Vorteil, wenn die Sensorfolie bzw. der Meßbereich vor der eigentlichen Messung auf eine konstante Temperatur thermostatisiert werden muß, was einige Minuten dauern kann.

In einer ersten Ausführungsvariante der Erfindung kann der Applikatortubus zur Thermostatisierung des Sensorfilms Öffnungen für die Zu- und Abfuhr eines Wärmeträgermediums, vorzugsweise Warmluft, aufweisen. Durch die Erwärmung des Hautbereiches auf Temperaturen > 33°C, vorzugsweise 37°C. wird die Aktivität der Haut gesteigert. Dadurch wird jene Zeit verkürzt, die abzuwarten ist, bis die Sauerstoffkonzentration an der Grenzfläche Haut/Sensorfilm mit der O₂-Konzentration subcutaner Gewebeschichten in das Gleichgewicht kommt. Erst nach dieser Einstell- bzw. Wartezeit mißt der Sensor jene Sauerstoffkonzentration, die dem subcutanen Sauerstoffpartialdruck entspricht. Durch die Maßnahme der Thermostatisierung wird die Vorbereitungszeit für die eigentliche Messung verkürzt, was sowohl für den Patienten als auch für das klinische Personal von Vorteil ist. Durch die Verwendung des erfindungsgemäßen Applikatortubus ist der Patient während der Einstell- bzw. Wartezeit nicht mit der Anregungs- und Detektionseinheit verbunden, wodurch sich der Patient im wesentlichen uneingeschränkt bewegen kann. Die Anregungs- und Detektionseinheit der Vorrichtung braucht für die eigentliche Messung nur kurzfristig mit der Anschlußvorrichtung des Applikatortubus verbunden werden.

Erfindungsgemäß ist es auch möglich, daß der Applikatortubus zur Thermostatisierung des Sensorfilms eine Strahlungs- oder Heizeinrichtung, beispielsweise eine IR-Lichtquelle oder eine elektrische Heizwendel aufweist.

Im Zusammenhang mit dem beschriebenen Applikatortubus ist der flächige Sensorfilm keinerlei Einschränkungen unterworfen. Es kann sich dabei um eine an der Meßoberfläche auf-bzw. anbringbare Sensorpaste, Sensorfolie oder aufgesprühte Schicht handeln.

Die erfindungsgemäße Vorrichtung eignet sich bei ansonsten völlig identer Bauweise sowohl zur Konzentrations- oder Partialdruckmessung als auch zur Flußmessung, wobei lediglich unterschiedliche Sensorfilme verwendet werden. So wird für die Konzentrations- oder Partialdruckmessung ein Sensorfilm verwendet, welcher an der der Meßoberfläche abgewandten Seite eine für den zu messenden Parameter undurchlässige Sperrschicht aufweist. Hingegen wird für die Flußmessung ein Sensorfilm verwendet, dessen allfällige zusätzliche Träger- und Füllschichten aus Materialien bestehen, welche für den zu messenden Parameter einen bekannten, endlichen Durchtrittswiderstand aufweisen.

In einer vorteilhaften Ausgestaltung der Erfindung ist vorgesehen, daß zwischen der Sensorfolie und der Meßoberfläche eine den Spalt zwischen Sensorfolie und Meßoberfläche überbrückende flexible Schicht, vorzugsweise eine Klebeschicht angeordnet ist, welche für den zu messenden Parameter permeabel ist. Durch diese Maßnahmen können Querdiffusionen des zu messenden Parameters gegenüber der raschen Diffusion in einem Luftspalt wirksam reduziert und die örtliche Auflösung der Messung erhöht werden. Weiters ist es von Vorteil, wenn die flexible Schicht zwischen Sensorfolie und Meßoberfläche für die Detektionseinrichtung störende Strahlung (z. B. Eigenfluoreszenz der Haut) undurchlässig ist.

Um ein Ausrichten der Anregungs- und Detektionseinheit zu ermöglichen, ist diese um ihre otpische Achse verdrehbar auf die Anschlußvorrichtung des Applikatortubus aufsetzbar.

Insbesondere bei der Anwendung der Vorrichtung an Armen und Beinen von Patienten ist der Applikatortubus mittels einer Manschette am Meßort fixierbar.

Für die Messung der Sauerstoffkonzentration oder des Sauerstofflusses ist im Sensorfilm vorzugsweise ein Lumineszenzindikator immobilisiert bzw. der Sensorfilm mit einem Lumineszenzindikator beschichtet, dessen örtliche Lumineszenzlöschung eine eindeutige Funktion der örtlichen Sauerstoffkonzentration ist.

Die Erfindung wird im Folgenden anhand von Zeichnungen näher erläutert.

Es zeigen:
- Fig. 1: die erfindungsgemäße Vorrichtung teilweise axial geschnitten,
- Fig. 2: den Applikatortubus der erfindungsgemäßen Vorrichtung samt abnehmbarem Deckel,
- Fig. 2A und 2B: zwei Ausführungsvarianten des bei der erfindungsgemäßen Vorrichtung verwendeten Sensorfilms jeweils in einer Schnittdarstellung sowie
- Fig. 3: die erfindungsgemäße Vorrichtung während der Meßphase.

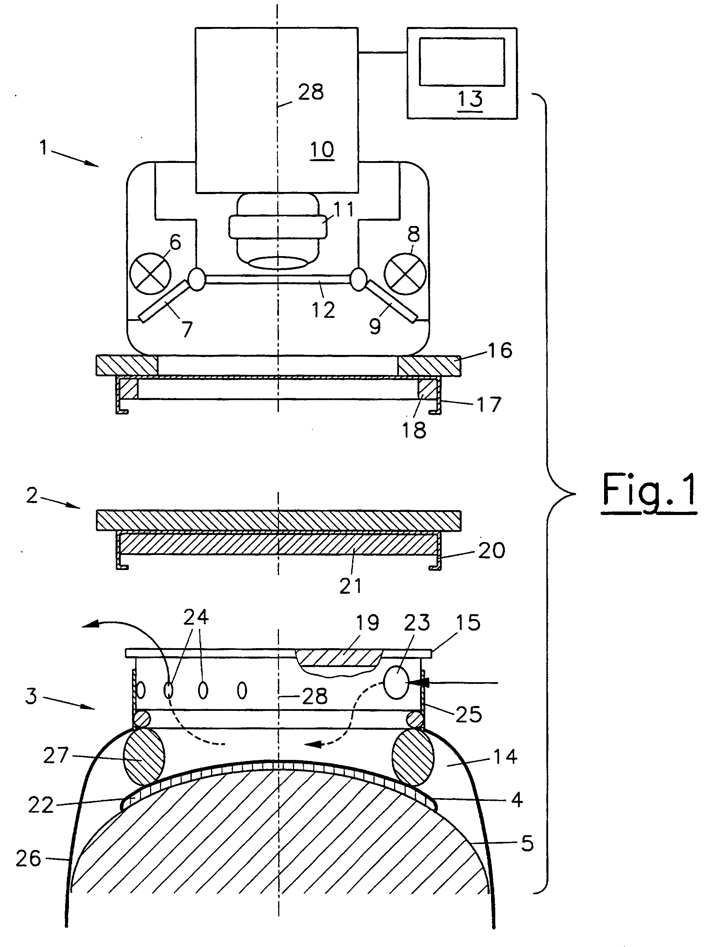

Die in Fig. 1 dargestellte Ausführungsvariante der erfindungsgemäßen Vorrichtung zur quantitativen Bestimmung der örtlichen Verteilung einer Meßgröße ist im wesentlichen dreiteilig aufgebaut und besteht aus einer Anregungs- und Detektionseinheit 1, einem abnehmbaren Deckel 2 und einem Applikatortubus 3. Weiters weist die Vorrichtung einen flächigen Sensorfilm 4 auf, welcher auf eine gekrümmte Meßoberfläche 5, beispielsweise einem Hautbereich am Arm eines Patienten, anbringbar ist. Der Sensorfilm 5 weist einen Lumineszenzindikator auf, welcher auf den zu bestimmenden Parameter mit einer Änderung zumindest einer optischen Eigenschaft reagiert. Die Anregungs- und Detektionseinheit 1 weist eine Einrichtung zur Bereitstellung einer Anregungsstrahlung zumindest einer Wellenlänge auf, wobei beispielsweise eine Lichtquelle 6 und ein Anregungsfilter 7 zur Bereitstellung einer Anregungsstrahlung der Wellenlänge λ₁ sowie eine Lichtquelle 8 und ein Anregungsfilter 9 zur Bereitstellung einer Anregungsstrahlung mit der Wellenlänge λ₂ vorgesehen sein können. Die bildgebende Detektionseinrichtung 10 weist z. B. eine CCD-Camera mit einer abbildenden Optik 11 sowie ein Emissionsfilter 12 auf. Die Detektionseinrichtung steht mit einer hier nur schematisch dargestellten Auswerteeinrichtung 13 zur Aufbereitung der durch die Anregungs- und Detektionseinrichtung 1 gewonnenen Bildinformation in Verbindung.

Die Auswerteeinheit 13 enthält beispielsweise Mittel zur Bildverarbeitung und Archivierung, Aufbereitung der Information (z. B. Darstellung als Falschfarbenbild, Histogramme etc.)

Der auf die Meßoberfläche 5 aufsetzbare Applikatortubus 3 weist einen der Meßoberfläche zugewandten Rand 14 auf, welcher elastisch am Sensorfilm 4 anliegt und für Störstrahlung undurchlässig ist. Das der Meßoberfläche 5 abgewandte Ende des Applikatortubus 3 weist eine Anschlußvorrichtung 15 für die Anregungs- und Detektionseinheit 1 auf.

Der abnehmabre Deckel 2 ist in jenen Meßsituationen von Vorteil, wo die Sensorfolie 4 bzw. der davon bedeckte Hautbereich vor der eigentlichen Messung thermostatisiert werden müssen. In Meßsituationen ohne vorangehender Thermostatisierung kann der Deckel 2 entfallen, und die Anregungs- und Detektionseinheit 1 direkt auf den Applikatortubus 3 aufgesetzt werden, wobei der an einem Auflagering 16 angeordnete Verschluß 17 (beispielsweise Bajonettverschluß) in der Anschlußvorrichtung 15 des Applikatortubus 3 einrastet. Ein Dichtring 18 am Auflagering 16 dient zur lichtdichten Ankoppelung der Detektionseinheit.

Gemäß einer Ausführungsvariante kann das von der Meßoberfläche 5 abgewandte Ende des Applikatortubus 3 auch mit einer für die Anregungs- und Meßstrahlung durchlässigen Abdeckung 19, beispielsweise mit einer Glasplatte abgeschlossen sein, wodurch der vom Applikatortubus 3 eingeschlossene Raum ebenfalls thermostatisierbar ist und auf den abnehmbaren Deckel 2 verzichtet werden kann. Der abnehmbare Deckel 2 weist den gleichen Verschluß 20, wie die Anregungs- und Detektionseinheit 1 auf, und kann an der Innenseite eine Dicht- bzw. Isolationsschicht 21 aufweisen.

Zwischen der Sensorfolie 4 und der Meßoberfläche 5 ist eine den Spalt zwischen Sensorfolie und Meßoberfläche überbrückende flexible Schicht 22 angeordnet, welche für den zu messenden Parameter permeabel ist. Da sowohl die Sensorfolie 4 als auch die flexible Schicht 22 sehr dünn ausgeführt werden können, kann die lokale Sauerstoffkonzentration im Sensorfilm mit hoher örtlicher Auflösung bestimmt werden, wobei die örtliche Differenzierung im wesentlichen durch die Diffusionseigenschaften der Haut und nicht durch die Meßeinrichtung limitiert wird.

Um die Genauigkeit und Reproduzierbarkeit der Messung zu optimieren, weist der Applikatortubus 3 Mittel zur Thermostatisierung des Sensorfilms 4 sowie des darunterliegenden Hautbereiches auf. Beispielsweise können diese Mittel Öffnungen für die Zufuhr 23 und die Abfuhr 24 eines Wärmeträgermediums, vorzugsweise Warmluft, umfassen.

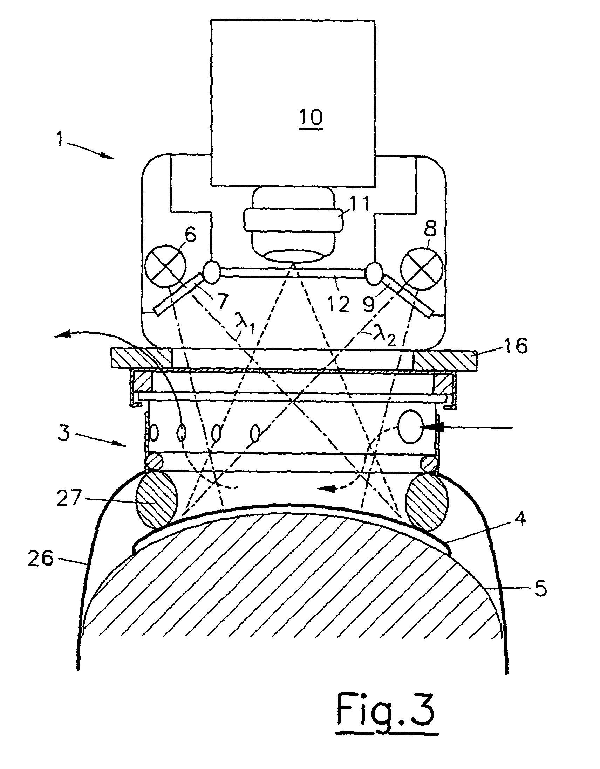

Der Indiaktortubus 3 kann vorzugsweise zur Anbringung an Armen und Beinen einen Stützring 25 aufweisen, welcher mit Hilfe einer Manschette 26 auf der Extremität befestigt werden kann. Der der Meßoberfläche 5 bzw. der Sensorfolie 4 zugwewandte elastische Rand des Applikatortubus 3 kann als Auflagering 27 aus einem elastischen Material, wie z. B. Schaumstoff, Moosgummi, einem gasgefüllten Schlauch oder ähnlichem hergestellt sein. Der Auflagering 27 begrenzt das Detektionsfeld der Detektionseinrichtung 10 (siehe Fig. 3). Er weist einen Durchmesser auf, welcher kleiner ist, als die applizierte Sensorfolie, weil deren Randbereiche nur begrenzt für die Messung nutzbar sind und der Auflagering auch teilweise die Sensorfolie fixieren kann. Der elastische Auflagering 27 wirkt auch als Dichtung für die Luftheizung.

Der Applikatortubus 3 stellt mit seinem Stützring 25 und der Befestigungsmanschette 26 den in bezug zum Sensorfilm 4 unbeweglichen Teil der Meßvorrichtung dar. Er dient als Auflager für den Optiktubus der Anregungs- und Detektionseinheit, welche um ihre optische Achse 28 verdrehbar auf die Anschlußvorrichtung 15 des Applikatortubus 3 aufsetzbar ist. Dadurch kann die CCD-Kamera in die gewünschte Position gebracht werden.

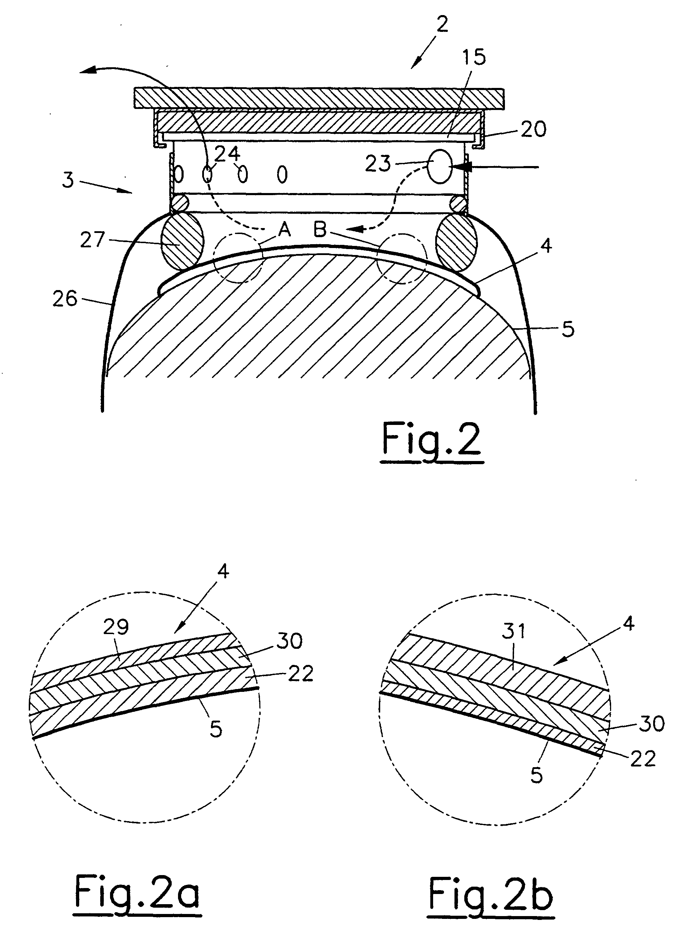

In der Abbildung gemäß Fig. 2 ist der Applikatortubus 3 während der Thermostatisierung durch den abnehmbaren Deckel 2 verschlossen. Die Verschlußelemente 20 des Deckels 2 sind dabei auf die Anschlußvorrichtung 15 des Applikatortubus 3 aufgesetzt. Durch die Einlaßöffnung 23 gelangt das Wärmeträgermedium, beispielsweise Warmluft, in den Tubus und kann durch Auslaßöffnungen 24, welche so angeordnet sind, daß eine gleichmäßige Durchströmung des Volumens gewährleistet ist, den Tubus wieder verlassen. Die Auslaßöffnungen 24 müssen beispielsweise durch Filterelemente lichtdicht ausgeführt sein. Weiters ist ein hier nicht dargestellter Temperaturfühler vorgesehen, welcher mit einer externen Heiz- und Gebläseeinrichtung einen geschlossenen Regelkreis bildet. Damit kann eine temperaturstabile Erwärmung des Sensorfilms und des darunterliegenden Hautbereiches auf 33°C bis 37°C erzielt werden. Weiters kann ein mit der Auswerteeinheit verbundener Temperaturfühler zur Überwachung der Tubustemperatur vorgesehen sein.

Wie bereits in Fig. 1 als Variante dargestellt, kann der Applikatortubus 3 auch mit einer transparenten Glasplatte 19 verschlossen sein. Bei dieser Ausführungsvariante wird auf den abnehmbaren Deckel 2 verzichtet. Nachteilig bei der Verwendung einer Glasplatte können allerdings Reflexionen des Anregungs- oder Fluoreszenzlichtes sein. Sowohl die Ausführung mit abnehmbaren Deckel 2 als auch jene mit einer transparenten Abdeckung 19 weist den Vorteil auf, daß während der Vorwärmphase die Anregungs- und Detektionseinrichtung 1 abgenommen werden kann, wodurch der Patient vom Gewicht der Kamera entlastet wird. Weiters kann die Anregungs- und Detektionseinheit 1 während der Vorwärmephase für andere Patienten verwendet werden.

In den Fig. 2a bzw. 2b sind Details der Varianten A bzw. B des Sensorfilms 4 vergrößert dargestellt. In beiden Fällen ist direkt auf der Meßoberfläche 5, beispielsweise der Haut, eine flexible Schicht 22 vorgesehen, welche Bestandteil des Sensorfilms sein kann oder auch eine Kleberschicht, welche zur Überbrückung des Spaltes zwischen Meßoberfläche und Sensorfilm direkt auf die Haut aufgetragen wird. Die Schicht ist vorzugsweise als optische Isolationsschicht ausgeführt. Die Schicht muß allerdings für den zu messenden Parameter durchlässig sein. Für die Messung der transcutanen Sauerstoffkonzentration weist der in Fig. 2a dargestellte Sensorfilm an der der Meßoberfläche 5 abgewandten Seite eine für den zu messenden Parameter undurchlässige Sperrschicht 29 auf. Die Indikatorschicht ist mit 30 bezeichnet.

Für die Messung des Sauerstoffflusses weist der Sensorfilm 4 gemäß Fig. 2b neben der eigentlichen Indikatorschicht 30 eine Trägerschicht 31 auf, welche aus einem Material besteht, das für den zu messenden Parameter einen bekannten, endlichen Durchtrittswiderstand aufweist. Es ist allerdings auch möglich, die Indikatorschicht 30 aus einem Matrixmaterial herzustellen bzw. mit Füllschichten zu versehen, welche eine Diffusionsbarriere für den zu messenden Parameter, beispielsweise Sauerstoff, darstellen.

In Fig. 3 ist die erfindungsgemäße Vorrichtung in der Meßphase dargestellt. Zu diesem Zweck wird die Anregungs- und Detektionseinheit 1 auf den Applikatortubus 3 aufgesetzt. In der Einheit 1 sind eine oder mehrere Lichtquellen 6, 8 so angeordnet, daß eine möglichst homogene Ausleuchtung des Sensorfilms 4 erzielt wird. Für eine Multiparameteranalyse können Lichtquellen unterschiedlicher Wellenlängen λ₁ und λ₂ (z. B. λ₁ = 450nm, λ₂ = 525 nm) eingesetzt werden. Für jede Lichtquelle 6. 8 oder jedes Lichtquellenarray kann bei Bedarf ein passender optischer Anregungsfilter 7, 9 im Strahlengang positioniert werden. Mit den Anregungsfiltern werden unerwünschte Strahlungsanteile des Anregungslichtes ausgefiltert. Das Emissionsfilter 12 vor der fokussierenden Optik 11 dient dazu, rückgestreutes oder reflektiertes Anregungslicht von der Detektionseinrichtung 10 fernzuhalten und nur das vom Sensorfilm 4 emittierte Fluoreszenzlicht durchzulassen. Die fokussierende Optik 11 kann eine Zoom-Funktion enthalten, um die lokale Auflösung der Bildinformation zu verändern.

Im Vergleich zur bisher etablierten Methode der punktweisen O₂-Partialdruck-Messung mittels einer Miniaturelektrode ergeben sich folgende Vorteile mit der erfindungsgemäßen Vorrichtung.
- Wesentlich genaueres und besser reproduzierbares Meßverfahren, da der Fluoreszenzindikator des Sensorfilms keinen Sauerstoff verbraucht, sondern ausschließlich auf dessen lokale Konzentration reagiert.
- Unabhängig von elektromagnetischen Feldern.
- Möglichkeit der Bestimmung des nutritiven Sauerstoffgehaltes der Haut als Hilfe für Therapieentscheidungen.
- Kurzer und schmerzloser Meßvorgang am Patienten.
- Physiologisch unproblematische Meßtemperaturen (max. 37°C).
- Nichtinvasives Meßverfahren.
- Hygienisches Meßverfahren, da nur der Sensorfilm, der nach der Messung entsorgt wird, direkt mit den Hautstellen des Patienten in Kontakt kommt.
- Erfassung und ortsaufgelöste Darstellung der Meßgröße über einen Bereich von ca. 5 x 4 cm der Haut mit nur einem Meßvorgang.
- Anwendung für flächige Messungen an gekrümmten Oberflächen.
- Geringe Meßdauer (< 5 sek).
- Eindeutige Zuordnung der gemessenen O₂-Verteilung zu den entsprechenden Hautstellen.
- Durch Bestimmung der Meßwertverteilungen: Heterogenität der Meßwerte kann erfaßt und zu statistischer Analyse genutzt werden.
- Flächiger Überblick ist wesentlich repräsentativer als eine Punktmessung.
- Möglichkeit der gesteigerten Auflösung durch Änderung der Abbildungsgeometrie.
- Möglichkeit der softwaregestützten Datenauswertung und graphischen Darstellung am Monitor, wobei an bestimmten Meßpunkten (beispielsweise der Courser-Position) der Meßwert oder dessen Gradient agezeigt werden kann, weiters Falschfarbendarstellung, Minima/Maxima, Histogramme, Archivierung.
- Einfache Handhabung.

## Patentansprüche

1. Vorrichtung zur quantitativen Bestimmung der örtlichen Verteilung einer Meßgröße, mit einem auf eine Meßoberfläche (5), beispielsweise eine Organoberfläche, einen Hautbereich oder eine Zellkultur, aufbringbaren flächigen Sensorfilm (4) mit bekannten Diffusionseigenschaften für einen zu bestimmenden Parameter, wobei der Sensorfilm (4) einen auf den zu bestimmenden Parameter mit einer Änderung zumindest einer optischen Eigenschaft reagierenden Lumineszenzindikator aufweist, mit einer Anregungs- und Detektionseinheit (1), welche eine Einrichtung (6, 7, 8, 9) zur Bereitstellung einer Anregungsstrahlung zumindest einer Wellenlänge und eine bildgebende Detektionseinrichtung (10, 11, 12), vorzugsweise eine CCD-Kamera aufweist, sowie mit einer Auswerteeinrichtung (13) zur Aufbereitung der durch die Anregungs- und Detektionseinrichtung (1) gewonnenen Bildinformation, **dadurch gekennzeichnet, daß** die Vorrichtung einen auf die Meßoberfläche (5) aufsetzbaren Applikatortubus (3) aufweist, dessen der Meßoberfläche (5) zugewandte Rand (14) am Sensorfilm (4) elastisch anliegt und für die Detektionseinrichtung störende Strahlung undurchlässig ist.

2. Vorrichtung nach Anspruch 1, **dadurch gekennzeichnet, daß** der flächige Sensorfilm (4) eine flexible, an der Meßoberfläche (5) auf- bzw. anbringbare Sensorpaste, Sensorfolie oder aufgesprühte Schicht ist.

3. Vorrichtung nach Anspruch 1 oder 2, **dadurch gekennzeichnet, daß** der an der Meßoberfläche (5) anliegende Sensorfilm (4) an der der Meßoberfläche (5) abgewandten Seite eine für den zu messenden Parameter undurchlässige Sperrschicht (29) aufweist.

4. Vorrichtung nach Anspruch 1 oder 2, **dadurch gekennzeichnet, daß** der an der Meßoberfläche (5) anliegende Sensorfilm (4) sowie allfällige zusätzliche Träger- und Füllschichten (31) aus Materialien bestehen, welche für den zu messenden Parameter einen bekannten, endlichen Durchtrittswiderstand aufweisen.

5. Vorrichtung nach einem der Ansprüche 1 bis 4, vorzugsweise zur Anwendung an gekrümmten Meßoberflächen (5), **dadurch gekennzeichnet, daß** die Detektionseinrichtung (10) eine Einheit zur Bestimmung der örtlichen Fluoreszenzabklingzeit oder einer davon abgeleiteten Größe aufweist.

6. Vorrichtung nach einem der Ansprüche 2 bis 4, **dadurch gekennzeichnet, daß** zwischen der Sensorfolie (4) und der Meßoberfläche (5) eine den Spalt zwischen Sensorfolie (4) und Meßoberfläche (5) überbrückende flexible Schicht (22), vorzugsweise eine Klebeschicht angeordnet ist, welche für den zu messenden Parameter permeabel ist.

7. Vorrichtung nach Anspruch 6, **dadurch gekennzeichnet, daß** die flexible Schicht (22) für die Detektionseinrichtung störende Strahlung undurchlässig ist.

8. Vorrichtung nach einem der Ansprüche 1 bis 7, **dadurch gekennzeichnet, daß** der der Meßoberfläche (5) zugewandte Rand des Applikatortubus (3) einen elastischen Auflagering (27) aufweist.

9. Vorrichtung nach einem der Ansprüche 1 bis 8, **dadurch gekennzeichnet, daß** der Applikatortubus (3) an dem der Meßoberfläche (5) abgewandten, verschließbaren Ende eine Anschlußvorrichtung (15) für die Anregungs- und Detektionseinheit (1) aufweist.

10. Vorrichtung nach einem der Ansprüche 1 bis 9, **dadurch gekennzeichnet, daß** der Applikatortubus (3) am von der Meßoberfläche (5) abgewandten Ende mit einer für die Anregungs- und Meßstrahlung durchlässigen Abdeckung (19), vorzugsweise einer Glasplatte abgeschlossen, oder mit einem abnehmbaren Deckel (2) verschließbar ist.

11. Vorrichtung nach Anspruch 9 oder 10, **dadurch gekennzeichnet, daß** die Anregungs- und Detektionseinheit (1) um ihre optische Achse (28) verdrehbar auf die Anschlußvorrichtung (15) des Applikatortubus (3) aufsetzbar ist.

12. Vorrichtung nach einem der Ansprüche 1 bis 11, **dadurch gekennzeichnet, daß** der Applikatortubus (3) zur Thermostatisierung des Sensorfilms (4) Öffnungen (23, 24) für die Zu- und Abfuhr eines Wärmeträgermediums, vorzugsweise Warmluft, aufweist.

13. Vorrichtung nach einem der Ansprüche 1 bis 11, **dadurch gekennzeichnet, daß** der Applikatortubus (3) zur Thermostatisierung des Sensorfilms (4) eine Strahlungs- oder Heizeinrichtung. beispielsweise eine IR-Lichtquelle oder eine elektrische Heizwendel aufweist.

14. Vorrichtung nach einem der Ansprüche 1 bis 13, **dadurch gekennzeichnet, daß** der Applikatortubus (3) mittels einer Manschette (26) am Meßort fixierbar ist.

15. Vorrichtung nach einem der Ansprüche 1 bis 14, **dadurch gekennzeichnet, daß** im Sensorfilm (4) ein Lumineszenzindikator immobilisiert ist oder daß der Sensorfilm (4) mit einem Lumineszenzindikator beschichtet ist, dessen örtliche Lumineszenzlöschung eine eindeutige Funktion der örtlichen Sauerstoffkonzentration ist.

## Claims

1. A system for quantitative determination of the local distribution of a quantity to be measured, including a planar sensor film (4), which is applied to a possibly curved measuring surface (5), such as the surface of a body organ, a skin area or a cell culture, and whose diffusion properties regarding a parameter to be determined are known, said sensor film (4) containing a luminescent indicator responding to the parameter to be determined with a change of at least one optical property, comprising an excitation and detection unit (1) including a means (6,7,8,9) for supplying excitation radiation of at least one wavelength and an imaging detection means (10,11,12), preferably a CCD camera, and an evaluation unit (13) processing the imaged information obtained from the excitation and detection unit (1) **characterized in that** is further provided an applicator tube (3) to be placed on the measuring surface (5), whose rim (14) facing the measuring surface (5) is in elastic contact with the sensor film (4) and is impervious to external radiation interfering with the detection means.

2. A system as claimed in 1 **characterized in that** the planar sensor film (4) applied to the measurement surface (5) is a sensor paste or a sensing foil or a spray layer.

3. A system as claimed in 1 or 2 **characterized in that** the sensorfilm (4) adjacent to the measurement surface (5) has, on its side facing away from the measurement surface (5), a barrier layer (29) that is impermeable to the parameter to be determined.

4. A system as claimed in 1 or 2 **characterized in that** the sensorfilm (4) adjacent to the measurement surface (5) and other supporting and filling layers (31) consist of materials which exhibit a known, finite resistance to the passage of the parameter to be determined.

5. A system as claimed in one of claims 1 to 4, preferably used for curved measurement surfaces (5), **characterized in that** the detection means (10) comprise a unit for determining the local fluorescence decay time or a derived quantity.

6. A system as claimed in one of claims 2 to 4, **characterized in that** a flexible layer (22) is provided between the sensor film (4) and the measurement surface (5) bridging the gap between sensor film and measuring surface, preferably an adhesive layer which is permeable to the parameter to be determined.

7. A system as claimed in 6, **characterized in that** the flexible layer (22) is impervious to radiation interfering with the detection unit.

8. A system as claimed in one of claims 1 to 7, **characterized in that** the rim of the applicator tube (3) facing the measurement surface (5) is provided an elastic supporting ring (27).

9. A system as claimed in one of claims 1 to 8, **characterized in that** the applicator tube (3) is provided at its coverable end facing away from the measuring surface (5) with a coupling means (15) for attaching the excitation and detection unit.

10. A system as claimed in one of claims 1 to 9, **characterized in that** the applicator tube (3) may be covered at the end facing away from the measurement surface (5) by a cover (19) that is transparent to the excitation and measurement radiation, preferably a glass plate, or by a removeable cap (2).

11. A system as claimed in one of claims 9 or 10, **characterized in that** the excitation and detection unit (1) can be attached to the coupling means (15) of the applicator tube (3) so as to be rotatable about its optical axis (28).

12. A system as claimed in one of claims 1 to 11, **characterized in that** the applicator tube (3) is provided with inlet and outlet openings (23, 24) trough which a heat transfer medium, preferably hot air, is introduced and carried off for thermoregulation of the sensor film (4).

13. A system as claimed in one of claims 1 to 11, **characterized in that** the applicator tube (3) is provided with a radiation- or heating element for thermoregulation of the sensor film (4), such as an infrared light source or an electric heater coil.

14. A system as claimed in one of claims 1 to 13, **characterized in that** the applicator tube (3) may be held in place at the measurement site by means of a cuff.

15. A system as claimed in one of claims 1 to 14, **characterized in that** a luminescent indicator is immobilized in the sensor film, (4) or the sensor film (4) is coated with a luminescent indicator, whose local luminescence quenching is a unique function of the local oxygen concentration.

## Revendications

1. Dispositif de détermination quantitative de la répartition locale d'une grandeur de mesure, comprenant :
- un film sensible en surface (4) applicable sur une surface à mesurer (5), par exemple une surface d'organe, une partie de peau ou une culture de cellule, ce film (4) ayant des caractéristiques de diffusion connues pour un paramètre à définir, et comportant un indicateur de luminescence réagissant à un paramètre à déterminer, par variation d'au moins une propriété optique,
- une unité d'excitation et de détection (1) composée d'une installation (6, 7, 8, 9) fournissant un rayonnement d'excitation d'au moins une longueur d'onde et une installation de détection (10, 11, 12) formant une image, de préférence une caméra CCD, et
- une installation d'exploitation (13) pour traiter une information d'image obtenue par l'installation d'excitation et de détection (1),
**caractérisé en ce que**
le dispositif comporte un tube applicateur (3) pouvant se placer sur la surface à mesurer (5) et dont le bord (14) tourné vers la surface à mesurer (5) est appliqué élastiquement contre le film sensible (4) et est imperméable aux rayonnements perturbateurs pour l'installation de détection.

2. Dispositif selon la revendication 1,
**caractérisé en ce que**
le film sensible en surface (4) est une pâte sensible souple appliquée ou applicable sur la surface de mesure (5), ou une feuille sensible, ou une couche appliquée par pulvérisation.

3. Dispositif selon l'une quelconque des revendications 1 ou 2,
**caractérisé en ce que**
le film sensible (4), appliqué contre la surface (5) à mesurer, présente sur sa face non tournée vers la surface à mesurer (5), une couche barrière (29) imperméable au paramètre à mesurer.

4. Dispositif selon l'une quelconque des revendications 1 ou 2,
**caractérisé en ce que**
le film sensible (4) qui s'applique contre la surface à mesurer (5) ainsi que les couches de support ou de remplissage, supplémentaires (31) dans tous les cas, sont en des matières ayant une résistance de passage finie connue pour le paramètre à mesurer.

5. Dispositif selon l'une quelconque des revendications 1 à 4, appliqué notamment à des surfaces de mesure courbes (5),
**caractérisé en ce que**
l'installation de détection (10) comporte une unité pour déterminer le temps d'atténuation locale de fluorescence ou une grandeur qui en est déduite.

6. Dispositif selon l'une quelconque des revendications 2 à 4,
**caractérisé en ce qu'**
entre le film sensible (4) et la surface de mesure (5) on a une couche souple (22) qui recouvre l'intervalle entre la feuille sensible (4) et la surface de mesure (5), cette couche étant perméable au paramètre à mesurer et de préférence une couche adhésive.

7. Dispositif selon la revendication 6,
**caractérisé en ce que**
la couche souple (22) est imperméable à un rayonnement perturbant l'installation de détection.

8. Dispositif selon l'une quelconque des revendications 1 à 7,
**caractérisé en ce que**
le bord du tube applicateur (3) tourné vers la surface supérieure (5) à mesurer comporte un anneau d'appui élastique (27).

9. Dispositif selon l'une quelconque des revendications 1 à 8,
**caractérisé en ce que**
le tube applicateur (3) comporte, à son extrémité obturable non tournée vers la surface de mesure (5), un dispositif de raccordement (15) pour l'unité d'excitation et de détection (1).

10. Dispositif selon l'une quelconque des revendications 1 à 9,
**caractérisé en ce que**
le tube applicateur (3) peut être obturée, à son extrémité non tournée vers la surface de mesure (5), par un opercule (19) transparent au rayonnement d'excitation et de mesure, de préférence une plaque de verre, ou un couvercle amovible (2).

11. Dispositif selon l'une quelconque des revendications 9 ou 10,
**caractérisé en ce que**
l'unité d'excitation et de détection (1) est installée sur le dispositif de raccordement (15) du tube applicateur (3) en pouvant tourner autour de son axe optique (28).

12. Dispositif selon l'une quelconque des revendications 1 à 11,
**caractérisé en ce que**
le tube applicateur (3) comporte des ouvertures (23, 24) pour l'alimentation et l'évacuation d'un fluide caloporteur, notamment de l'air chaud, pour mettre en température (thermostater) le film sensible (4).

13. Dispositif selon l'une quelconque des revendications 1 à 11,
**caractérisé en ce que**
le tube applicateur (3) comporte une installation de rayonnement ou de chauffage, par exemple une source de lumière infrarouge ou une spire chauffante électrique pour mettre en température le film sensible (4).

14. Dispositif selon l'une quelconque des revendications 1 à 13,
**caractérisé en ce que**
le tube applicateur (3) se fixe à l'emplacement de mesure par un manchon (26).

15. Dispositif selon l'une quelconque des revendications 1 à 14,
**caractérisé en ce que**
dans le film sensible (4) on immobilise un indicateur de luminescence, ou le film sensible (4) est revêtu d'un indicateur de luminescence dont l'extinction locale de luminescence est une fonction biunivoque vis-à-vis de la concentration locale en oxygène.
